# EUROPEAN PATENT SPECIFICATION

(11) **EP 1 505 950 B1**
(45) Date of publication and mention of the grant of the patent: **05.12.2007**
(21) Application number: 03731369.9
(22) Date of filing: 23.05.2003
(51) Int. Cl.: A61Q 11/00, A61K 6/00, A61K 8/34, A61K 8/21

(54) **ANTI-CARIES DENTAL VARNISH**
ZAHNLACK GEGEN KARIES
VERNIS DENTAIRE ANTI-CARIE

(30) Priority: 23.05.2002 US 382884 P
(43) Date of publication of application: 16.02.2005
(73) Proprietor: DENTSPLY INTERNATIONAL, INC., York, PA 17405-0872 (US)
(72) Inventor: SIMONTON, Thomas, C., Mount Wolf, PA 17347 (US); PETERSON, Kenneth, S., Lancaster, PA 17603 (US); SHERMAN, James, M., York, PA 17404 (US)
(74) Representative: Hartz, Nikolai
(86) International application number: PCT/US2003/016514
(87) International publication number: WO 2003/099245

(56) References cited:
- DE-A- 19 722 596

## Description

### TECHNICAL FIELD

The present invention is generally directed toward anti-caries dental materials. More particularly, the invention is directed toward such materials containing a sustained-release fluoride compound. Specifically, the invention relates to such materials containing a fluoride release controlling amount of glycerin and fumed silica.

### BACKGROUND OF THE INVENTION

The present invention relates to compositions for preventing dental caries. More particularly, it relates to fluoride-containing compositions that have enhanced activity in preventing dental caries. The use of soluble fluoride salts, such as stannous fluoride and about 5% by weight of sodium fluoride, to reduce the incidence of dental caries in the general population is a well-known and ongoing endeavor. The administration of these fluoride compounds takes many forms, including the fluoridation of drinking water, professional treatment by dentists and incorporation in oral hygiene compositions such as dentifrices and mouthrinses.

Of particular interest is the use of fluoride-containing varnish materials by dental professionals. Such materials are applied to the dentition and become adhered thereto. Over time, the fluoride is released, promoting remineralization of the tooth structure. Varnishes are also used to reduce tooth hypersensitivity.

Notwithstanding the widespread acceptance of such compositions, there is an ongoing search for more effective compositions and, therefore, there is a need to enhance the fluoride activity of various fluoride compounds by the addition of other compounds. For example, there has been suggested the use of various zinc salts to enhance the activity of fluoride compounds.

DE 197 22 596 A1 describes a dental varnish containing an alcohol, titanium dioxide, carboxymethylcellulose, a resin, and a fluoride. Journal of the American Dental Association 132 (2001) 1389-1392 evaluates fluoride release from commercially available dental fluoride varnishes.

### SUMMARY OF THE INVENTION

In general, a sustained-release, dental fluoride varnish material comprises about 5 percent by weight of sodium fluoride (NaF); from about 1 to about 10 percent by weight of glycerine; up to about 5 percent by weight of fumed silica; and optionally, an amount of varnish additives selected from the group consisting of flavorants, rosins, gums and alcohols.

Preferably, said varnish contains from about 1 to about 5 percent by weight of fumed silica.

### BRIEF DESCRIPTION OF THE DRAWING

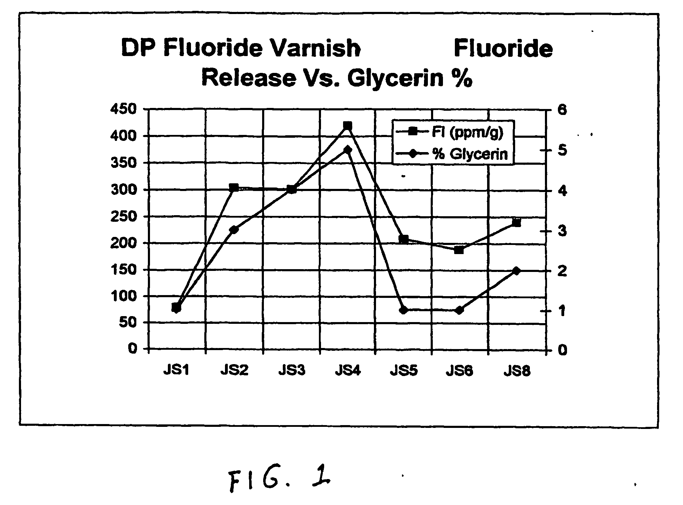

FIG. 1 is a graph depicting the fluoride release function of a number of inventive samples plotted versus the glycerin content thereof.

### PREFERRED EMBODIMENTS FOR CARRYING OUT THE INVENTION

An anti-caries dental varnish embodying the concepts of the present invention, contains about 5 percent by weight of NaF; from about 1 to about 10 percent by weight of glycerin; up to about 5 percent by weight of fumed silica; and an amount of varnish additives selected from the group consisting of flavorants, rosins, gums and alcohols. The inventive composition may also contain water.

The inventive fluoride varnish is a straw yellow to caramel brown, viscous material containing 5% sodium fluoride in a natural resin and alcohol/water base. The varnish is applied to a dry tooth by conventional techniques, and upon contact with saliva becomes adhered. Over time fluoride is released to the tooth surface promoting remineralization and desensitization.

Preferred rosins are those generally accepted for dental use, and include for example, vinsol rosin or wood rosin, both available from Hercules. The use of a rosin is optional, and the amount of such a rosin will vary depending upon the desired characteristics of the end product and upon the physical properties of the rosin itself.

Similarly, preferred gums include those generally accepted for dental use, and include for example, Portuguese gum available from Calo, Arentinian gum available from AKZO, and other gums such as Brazilian gum and the like. In a preferred embodiment of the invention, from about 50 to about 75 percent by weight (% W/W) is employed.

Similarly still, preferred alcohols include those generally accepted for dental use, and include for example, ethanol. From about 20 to 35 percent by weight of ethanol is preferred.

### GENERAL EXPERIMENTAL

As an example of a fluoride composition according to the present invention, the following material was prepared.

| **Addition Order** | **Ingredient** | **%W/W** | **Grams** | |
|---|---|---|---|---|
| | | **49** | **49-5x** | |
| 1 | Ethanol 190 proof | 24 | 120 | |
| | | | | |
| 2 | Portuguese Gum Calo | 63.5 | 317.5 | |
| | | | | |
| | | | | |
| | | | | |
| 3 | Glycerin | 5 | 25 | |
| 4 (vacuum mix) | Aerosil 200 | 1.5 | 7.5 | |
| 5 (vacuum mix) | NaF | 5 | 25 | |
| 6 | Flavor | 1 | 5 | |
| | | | | |
| | | | | |
| | | | | |
| | Total | 100 | 500 | |
| | | | | |
| | | | | |

As further examples, the dental varnish compositions as shown in TABLE I were also prepared.

**TABLE I**

| **Sample ID** | **JS1** | **JS2** | **JS3** | **JS4** | **JS5** | **JS6** | **JS8** |
|---|---|---|---|---|---|---|---|
| | **%W/W** | **%W/W** | **%W/W** | **%W/W** | **%W/W** | **%W/W** | **%W/W** |
| Ethanol 190 proof | 24 | 26 | 25 | 25 | 26 | 26 | 25 |
| Wood Rosin | 20 | 33 | 33 | 17.5 | 18 | 28 | 34 |
| Gum Rosin | 50 | 33 | 33 | 47.5 | 50 | 40 | 34 |
| Glycerin | 1 | 3 | 4 | 5 | 1 | 1 | 2 |
| Sodium Fluoride | 5 | 5 | 5 | 5 | 5 | 5 | 5 |
| | | | | | | | |

These samples, JS1-JSB, were then tested for fluoride release. The fluoride release data versus the amount of glycerin is plotted in FIG. 1. As can be seen in FIG. 1, the inventive composition shows improved fluoride release with even low amounts of glycerin, and shows even further improved released with higher amounts.

Another batch of samples were prepared as shown in TABLE II, each using Brazilian Gum in a dental varnish.

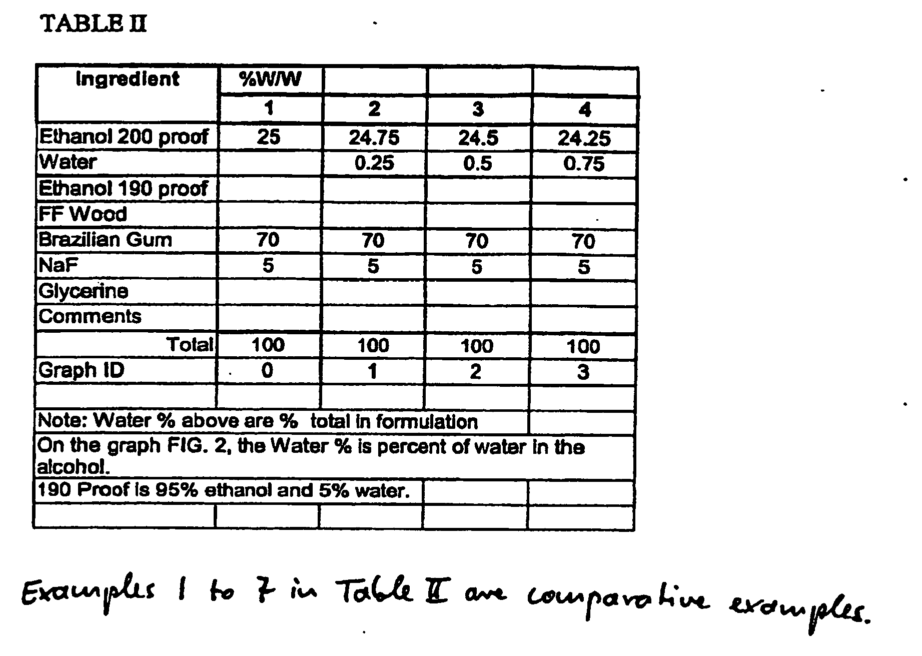

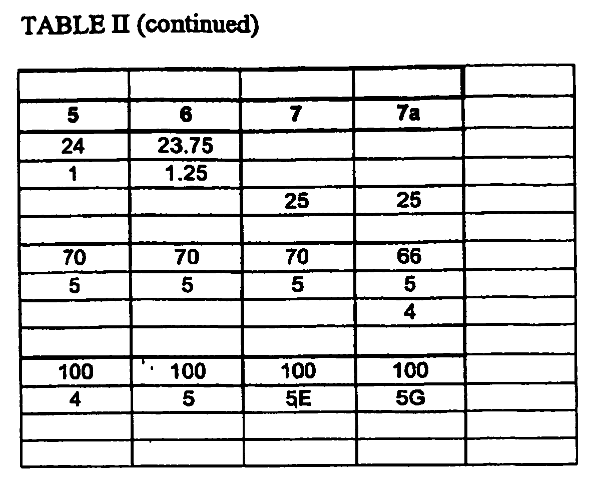

The test procedure for determining fluoride release showed amount of fluoride ion released to solution after 3 hours. For each test sample, three replicates are prepared. Sample cups were ¼ oz. white polypropylene with a 33mm opening and lid. (The manufacturer is Mold-Rite Plastics, NY.) Cup ID is 1/4oz 33mm RS PP wht. Each sample cup was numbered and weighed. Between 0.05g and 0.12 g of varnish were then painted around the inside of each cup, with the varnish confined to the bottom half of the cup. The varnish was painted uniformly without thick areas.

The painted cups were then reweighed immediately after varnish application, and then each cup was filled with distilled water and capped. Fluoride release was determined after three (3) hours. The total fluoride released is sensitive to contact time.

To determine fluoride release after 3 hours, the water was poured from the sample cups into separate small plastic beakers filled with 10ml of TISAB. After calibration a Fl meter was employed to measure and record Fl ion concentration for each sample. A prophy paste sample as a control to check calibration prior to measuring varnish sample was prepared according to test method GM-111-93. The ppm/g (parts per million per gram) released was determined by taking the fluoride reading in ppm and dividing by the varnish weight, taking into account any multipliers used during meter calibration.

It should be evident therefore, that a dental material such as a dental varnish, according to the present discussion, provides an improved anti-caries dental product, and otherwise is an advancement in the art. The invention has been exemplified herein without attempting to show all variations of the invention as to material component, grade, amount. The scope of the invention will therefore, be determined only by the claims.

## Claims

1. A sustained-release, dental fluoride varnish material comprising about 5 percent by weight of sodium fluoride (NaF); from about 1 to about 10 percent by weight of glycerin; up to about 5 percent by weight of fumed silica; and optionally, an amount of varnish additives selected from the group consisting of flavorants, rosins, gums and alcohols acceptable for dental use.

2. A varnish material as in claim 1, comprising about 5 percent by weight of said glycerin.

3. A varnish as in claim 1, comprising from about 33 to about 75 percent by weight of said gum.

4. A varnish as in claim 3, wherein said gum is selected from the group consisting of Portuguese gum and Brazilian gum.

5. A varnish as in claim 1, wherein said alcohol is present in an amount of from about 20 to 35 percent by weight of the varnish.

6. A varnish as in claim 4, wherein said alcohol is ethanol.

## Patentansprüche

1. Dentaler Fluoridlack mit Depotwirkung, umfassend ca. 5 Gew.-% Natriumchlorid (NaF), ca. 1 bis ca. 10 Gew.-% Glycerin, bis zu ca. 5 Gew.-% Quarzstaub, und gegebenenfalls Lackadditive, ausgewählt aus der Gruppe bestehend aus zur dentalen Verwendung annehmbaren Aromastoffen, Harzen (Rosins), Pflanzengummis und Alkoholen.

2. Lack nach Anspruch 1, umfassend ca. 5 Gew.-% Glycerin.

3. Lack nach Anspruch 1, umfassend ca. 33 bis ca. 75 Gew.-% Pflanzengummi.

4. Lack nach Anspruch 3, worin das Pflanzengummi ausgewählt ist aus der Gruppe bestehend aus portugiesischem Gummi (Portuguese Gum) und brasilianischem Gummi (Brazilian Gum).

5. Lack nach Anspruch 1, worin der Alkohol in einer Menge von ca. 20 bis 35 Gew.-% des Lacks vorhanden ist.

6. Lack nach Anspruch 4, worin der Alkohol Ethanol ist.

## Revendications

1. Matériau de vernis dentaire à base de fluorure à libération prolongée, comprenant environ 5 % en poids de fluorure de sodium (NaF) ; environ 1 à environ 10 % en poids de glycérol ; jusqu'à environ 5 % en poids de poudre de silice ultrafine ; et éventuellement une quantité d'additifs pour vernis choisis dans l'ensemble constitué d'agents aromatisants, de colophanes, de gommes et d'alcools acceptables pour une utilisation dentaire.

2. Matériau de vernis selon la revendication 1, comprenant environ 5 % en poids dudit glycérol.

3. Vernis selon la revendication 1, comprenant environ 33 à environ 75 % en poids de ladite gomme.

4. Vernis selon la revendication 3, dans lequel ladite gomme est choisie dans l'ensemble constitué de gomme du Portugal et de gomme du Brésil.

5. Vernis selon la revendication 1, dans lequel ledit alcool est présent en une quantité d'environ 20 à 35 % en poids du vernis.

6. Vernis selon la revendication 4, dans lequel ledit alcool est de l'éthanol.
